# EUROPÄISCHE PATENTANMELDUNG

(11) **EP 3 173 956 A1**
(43) Veröffentlichungstag der Anmeldung: **31.05.2017**
(21) Anmeldenummer: 15450041.7
(22) Anmeldetag: 24.11.2015
(51) Int. Cl.: G06F 19/00

(54) **COMPUTERIMPLEMENTIERTES VERFAHREN ZUR STEUERUNG EINES TRAININGSPROGRAMMS DURCH EINE BERÜHRUNGSSENTIVE OBERFLÄCHE**

(71) Anmelder: Walter, Christian, 1230 Wien (AT)
(72) Erfinder: Walter, Christian, 1230 Wien (AT)
(74) Vertreter: Keschmann, Marc

(57) **Zusammenfassung**

Bei einem computerimplementierten Verfahren zur Steuerung des Programmablaufs eines Trainingsprogramms, insbesondere für mentales Training, bei welchem der Programmablauf den Zeitpunkt und die Reihenfolge des Abspielens von elektronisch gespeicherten Sprachansagen bestimmt, werden periodische Bewegungen des Benutzers auf einer berührungssensitiven Oberfläche erfasst und ausgewertet und es wird der Programmablauf in Abhängigkeit vom Ergebnis der Auswertung gesteuert.

## Beschreibung

Die Erfindung betrifft ein computerimplementiertes Verfahren zur Steuerung des Programmablaufs eines Trainingsprogramms, insbesondere für mentales Training, bei welchem der Programmablauf den Zeitpunkt und die Reihenfolge des Abspielens von elektronisch gespeicherten Sprachansagen und Tonfolgen bestimmt.

Die Erfindung betrifft weiters ein Computerprogrammprodukt umfassend Programmcode-Mittel geeignet zur Durchführung der Schritte des erfindungsgemäßen Verfahrens, wenn das Computerprogrammprodukt auf einer Recheneinrichtung ausgeführt wird, sowie eine Recheneinrichtung, insbesondere Smartphone oder Tabletcomputer, zur Durchführung des erfindungsgemäßen Verfahrens.

Zum Erlernen und Üben verschiedenster Fähigkeiten werden Trainingsprogramme eingesetzt, die den Benutzer meist schrittweise durch die Trainingsinhalte leiten. Lern- oder Übungsinhalt wurden in der Vergangenheit hauptsächlich mit Hilfe von Lehrbüchern und/oder durch den persönlichen Vortrag des Lehrenden in Unterrichts- oder Übungseinheiten vermittelt. Daneben existieren gegenwärtig sogenannte eLearning-Angebote, bei denen im Audio- und/oder Video-Format aufgezeichnete Unterrichts- oder Übungseinheiten auf Ton- oder Videoträgern oder zum Herunterladen auf ein Datenverarbeitungsgerät zur Verfügung gestellt werden.

Im Audioformat bereitgestellte Trainingsprogramme können zur Vermittlung der verschiedensten Trainingsinhalte Verwendung finden, wie z.B. für Sprechtraining, körperliches Training oder mentales Training. Im Gegensatz zu multimedial aufbereiteten Trainingsinhalten, deren Wiedergabe über die graphische Benutzeroberfläche eines Personal Computers gesteuert werden kann und die daher oft interaktiv gestaltet sind, kommen lediglich im Audioformat bereitgestellte Trainingsprogramme ohne graphische Benutzeroberfläche aus und bieten daher nur begrenzte Möglichkeiten für interaktive Funktionen. Für die Audiowiedergabe vorgesehene Trainingsprogramme bestehen daher in der Regel aus Sprachansagen, die in einer vorgegebenen zeitlichen Reihenfolge abgespielt werden. Ein variabler Programmablauf, bei dem elektronisch gespeicherte Sprachansagen oder Tonsignale zu veränderbaren Zeitpunkten und in veränderbarer Reihenfolge abgespielt werden und der Zeitpunkt und die Reihenfolge des Abspielens automatisch in Abhängigkeit vom Verhalten des Benutzers opti-miert werden, ist meist nicht vorgesehen. Sofern eine Beein-flussungsmöglichkeit des Trainingsprogramms vorgesehen ist, beruht diese meist auf externen Sensoren für bestimmte Körperfunktionen, wie z.B. den Herzschlag oder die Atemfrequenz. Die Abspieleinrichtung zum Abspielen von elektronisch gespeicherten Sprachansagen muss in diesen Fällen daher aufwändig mit externen Sensoren ausgestattet werden.

Trainingsprogramme mit aufgesprochenen Übungen werden oft für Zwecke des Mentaltrainings angewendet. Mentaltraining ist die Durchführung von systematisch angeleiteten Übungen gemäß einer bekannten Mentaltrainingsmethode zur zweckorientierten Veränderung von Denkprozessen, Denkmustern oder Wahrnehmungen. Dadurch können, je nach Anforderung, etwa Stress oder Angstzustände reduziert, die körperliche oder die geistige Leistungsfähigkeit auf speziell erwünschten Gebieten erhöht, oder das allgemeine Wohlbefinden verbessert werden. Besonders in den westlichen Industrieländern besteht ständig wachsendes Interesse sowie Bedarf an der Anwendung von Mentaltrainingsmethoden. Wie bei jeder anderen Fertigkeit auch, erfordert ihr Erlernen allerdings methodische Kenntnisse und deren systematische Anwendung sowie Motivation und Durchhaltevermögen. Nicht nur autodidaktisch Übende, sondern auch alle Teilnehmer entsprechender Kurse für Mentaltraining müssen in der Lage sein, ihr regelmäßiges, meist täglich auszuführendes individuelles Übungspensum in den üblicherweise einwöchigen Zeitabständen zwischen trainerbegleiteten Übungseinheiten kontinuierlich, zielgerichtet und völlig eigenständig absolvieren zu können. Für ein zweckdienliches und an individuelle Bedürfnisse anpassbares Unterstützungsverfahren besteht daher weltweit ein großes gewerbliches Anwendungspotential.

Die übende Person kann beim Abspielen der Sprachansagen bzw. aufgesprochenen Übungen mithilfe der angeleitet erteilten Unterstützung den für Mentaltraining optimalen, entspannten und assoziationsbereiten Zustand, welcher auf neuronaler Ebene auf einer Aktivierung des Parasympathikus und einer Schwächung des Sympathikus beruht, üblicherweise leichter erreichen und einfacher erhalten. Sie kann aber den starr vorgegebenen Übungsverlauf anders als durch Stoppen oder durch Neupositionieren des Abspielvorgangs nicht beeinflussen. Durch derartige Maßnahmen würde allerdings der entspannte Trainingszustand unterbrochen und gestört. Durch abwechselnde Verwendung mehrerer, untereinander geringfügig unterschiedlicher, Tonaufnahmen kann zwar die sonst infolge von Gewöhnung unvermeidlich zunehmende und die Motivation hemmende Langeweile teilweise gemindert werden. Nachteilig bleibt jedoch in jedem Fall, dass der für Mentaltraining wichtige, und die Trainingsintensität zusätzlich verstärkende, Wirkkreislauf zwischen aktiven Rückmeldungen sowie unbewussten Körperreaktionen der übenden Person und die genau darauf abgestimmt erteilten neuerlichen Übungsanweisungen erst gar nicht in Gang kommen können.

Als nachteilig erweist sich bei audiobasierten Trainingsprogrammen somit, dass keine Beeinflussungsmöglichkeit des Abspielverlaufs möglich ist ohne dass das Training unterbrochen werden muss oder beeinträchtigt wird. Dadurch gelingt es der übenden Person im Normalfall nicht oder nicht ausreichend lange, die erforderliche Konzentration auf die Übungsinhalte zu richten.

Es ist daher wünschenswert, den Programmablauf so beeinflussen zu können, dass das Training nicht unterbrochen werden müss oder nicht beeinträchtigt wird. Die vorliegende Erfindung zielt daher darauf ab, eine technische Möglichkeit anzugeben, mit welcher der Programmablauf beeinflusst werden kann ohne dass die Abspieleinrichtung externe Sensoren für physiologische Messwerte erfordert.

Zur Lösung dieser Aufgabe sieht die Erfindung bei einem Verfahren der eingangs genannten Art im Wesentlich vor, dass periodische Bewegungen des Benutzers auf einer berührungssensitiven Oberfläche erfasst und ausgewertet werden und der Programmablauf in Abhängigkeit vom Ergebnis der Auswertung gesteuert wird. Dadurch, dass die Steuerung des Programmablaufs nicht durch punktuelle Benutzereingaben vorgenommen wird, sondern durch Erfassen einer periodischen Bewegung des Benutzers auf einer berührungssensitiven Oberfläche, kann beispielsweise ein gleichförmiger, wiederkehrender Bewegungsablauf erkannt werden, der den Trainingsablauf kaum beeinträchtigt und der ein minimales Maß an vom Trainingsinhalt wegführender Aufmerksamkeit erfordert. Weiters erhöht die periodische Bewegung auf Grund des repetitiven Charakters die Zuverlässigkeit der Erkennung derselben auf der berührungsempfindlichen Eingabeoberfläche. Unter einer periodischen Bewegung wird hierbei insbesondere eine sich wiederholende Bewegung oder ein sich wiederholendes Bewegungsmuster verstanden.

Die periodischen Bewegungen können beispielsweise Tipp-, Wisch- oder Ziehbewegungen des Benutzers auf der berührungssensitiven Oberfläche sein. Die periodischen Bewegungen werden vorzugsweise mit dem Finger des Benutzers ausgeführt. Eine bevorzugte Verfahrensweise sieht in diesem Zusammenhang vor, dass die periodische Bewegung eine hin und hergehende Wisch- oder Ziehbewegung ist. Die Hin- und Herbewegung, besonders beim Ziehen, hat den Vorteil, dass ein Aufheben des Fingers nicht erforderlich ist.

Gemäß einer besonders bevorzugten Ausbildung erfolgt die periodische Bewegung synchron mit dem Herzschlag oder der Atemfrequenz des Benutzers. Der Benutzer muss sich somit auf seinen Herzschlag und/oder auf seine Atemfrequenz konzentrieren und eine dem Herzschlag oder der Atemfrequenz synchrone Bewegung auf der berührungssensitiven Oberfläche durchführen. Dies fördert die insbesondere beim Mentaltraining anzustrebende Konzentration des Benutzers auf seine Körperfunktionen und erlaubt gleichzeitig eine von der jeweiligen Körperfunktion abhängige Steuerung des Programmablaufs ohne dass hierfür externe Sensoren erforderlich wären.

Die Erfindung kann mit entsprechend eingerichteten Datenverarbeitungsvorrichtungen durchgeführt werden, die über einen Speicher für die elektronisch gespeicherten Sprachansagen und Tonsignale, ein Abspielmodul zum Abspielen dieser Audiosignale über einen integrierten oder extern ankoppelbaren Lautsprecher sowie eine berührungssensitive Eingabeoberfläche verfügt. Die berührungssensitive Eingabeoberfläche kann beispielsweise als Touchpad ausgeführt sein. Eine bevorzugte Ausbildung sieht vor, dass die periodischen Bewegungen durch eine berührungssensitive elektronische Anzeigevorrichtung, insbesondere ein Touchdisplay, eines Computers, wie z.B. eines Smartphones, Tabletcomputers oder Personal Digital Assistent (PDA), erfasst werden. Ein Smartphone oder Tabletcomputer eignet sich besonders gut für die Realisierung der Erfindung, weil alle erforderlichen Hardwarekomponenten in integrierter Weise vorhanden sind. Die Erfassung und Auswertung der periodischen Bewegungen des Benutzers sowie die Steuerung des Programmablaufes können dabei in einer auf dem Smartphone bzw. dem Tabletcomputer ausführbaren Programmapplikation implementiert sein.

Um aus der periodischen Bewegung des Benutzers einen Steuerbefehl zur Änderung des Programmablaufs abzuleiten, sieht eine bevorzugte Verfahrensweise vor, dass das Auswerten der periodischen Bewegungen die Ermittlung einer Auswertungsgröße umfasst, wobei die Steuerung des Programmablaufs in Abhängigkeit von der Auswertungsgröße erfolgt.

Mit Vorteil wird als Auswertungsgröße die Häufigkeit der Bewegungen, die Anzahl von Bewegungen, die Geschwindigkeit der Bewegungen, die Frequenz der periodischen Bewegung, insbesondere der Hin- und Herbewegung, die Regelmäßigkeit der Bewegungen und/oder der zeitliche Abstand zwischen zwei aufeinanderfolgendem Bewegungen und/oder die Druckstärke ermittelt. Wenn die periodische Bewegung synchron mit dem Herzschlag oder der Atemfrequenz des Benutzers erfolgt, wird als Auswertungsgröße bevorzugt die Frequenz oder die Regelmäßigkeit des Herzschlags oder der Atmung ermittelt.

Die Beeinflussung des Programmablaufs in Abhängigkeit von der Auswertung der periodischen Bewegungen kann in unterschiedlichster Weise vorgenommen werden. Gemäß einer bevorzugte Ausführung ist vorgesehen, dass die Auswertungsgröße kontinuierlich oder in regelmäßigen Abständen ermittelt wird und mit wenigstens einem vorgegebenen -Grenzwert verglichen wird, wobei eine dem wenigstens einen vorgegebenen Grenzwert zugeordnete, gespeicherte Sprachansage und/oder ein entsprechendes Tonsignal bei Erreichen oder Unter- bzw. Überschreiten des Grenzwerts abgespielt wird. Dadurch kann der Programmablauf beispielsweise auf eine charakteristische Änderung der periodischen Bewegungen reagieren, in dem eine entsprechend angepasste Sprachansage abgespielt wird. Wenn die Bewegungen sich z.B. signifikant verlangsamen oder überhaupt aufhören, kann dies als Hinweis auf ein Abgleiten der Konzentration oder ein Einschlafen des Benutzers interpretiert werden, worauf eine geeignete Sprachansage auf ein Zurückholen oder Aufwecken des Benutzers gerichtet ist. Analog kann dadurch das Erreichen eines Entspannungszustandes des Benutzers erkannt werden, wenn die Frequenz der Atmung besonders regelmäßig ist oder zwischen Ein- und Ausatmungen bestimmte Zeitabstände vorliegen.

Besonders bevorzugt kann die Wiedergabegeschwindigkeit der Sprachansage bei gleichbleibender Tonhöhe in Abhängigkeit von der ermittelten Auswertungsgröße variiert werden. Auch hier kann im Zusammenhang mit der über die Bewegungen erfassten Atemfrequenz auf den mentalen oder körperlichen Zustand des Benutzers reagiert werden und bei Erkennen eines entspannten Zustands des Benutzers die Wiedergabegeschwindigkeit reduziert werden.

Alternativ oder zusätzlich kann vorzugweise so vorgegangen werden, dass die Pausenlänge zwischen dem Abspielen zweier aufeinanderfolgender Sprachansagen in Abhängigkeit von der ermittelten Auswertungsgröße gewählt wird.

Eine weitere denkbare Beeinflussung des Programmablaufs in Abhängigkeit von der erfassten Bewegungen kann bevorzugt dahingehend erfolgen, dass das Abspielen einer Sprachansage im Wesentlichen gleichzeitig mit dem Beginn der erfassten Bewegung, also z.B. beim Beginn des Einatmens oder beim Beginn des Ausatmens, gestartet wird.

In Bezug auf die Verwendung des erfindungsgemäßen Verfahrens für das Mentaltraining hat die Erfindung zusammengefasst die folgenden vorteilhaften Merkmale und Effekte.

Es können einerseits Finger-Tipp-Gesten des Benutzers auf der berührungssensitiven Eingabeoberfläche zur gezielten Steuerung des Übungsablaufs verwendet werden sowie zur Bestätigung bzw. Überprüfung der vollen persönlichen Bereitschaft zur Durchführung des nächsten Übungsschritts. An vorbestimmten Stellen des Programmablaufs können weiters Fragen angesagt werden, die durch wiederholtes Tippen mit einer definierten Anzahl von Tippbewegungen beantwortet werden können. Weiters wird dadurch die Möglichkeit zur Übungsablaufverzweigung nach spontanen persönlichen Bedürfnissen geschaffen. An wieder anderen, ebenfalls vorbestimmten Stellen können Verzweigungsmöglichkeiten angesagt werden: z.B. einmaliges Tippen bewirkt ein Springen zurück zum Anfang des Programms, ein zweimaliges Tippen bewirkt ein Springen zum Ende des Programms, etc.

Andererseits kann das Ausführen von der eigenen Atembeobachtung folgenden Bewegungsmustern mittels Fingerwisch- oder -ziehbewegungen zu einer Erhöhung der Konzentrationsfähigkeit durch bewusstes Wahrnehmen der eigenen Atemzüge über längere Zeiträume hinweg führen. Die Überlagerung von zwei Modalitäten (Ziehbewegungen des Fingers und Beobachtung der Atembewegung) hat eine ähnlich verstärkende Wirkung wie beim handschriftlichen Schreiben, wodurch die Merkfähigkeit erhöht wird.

Die ausgeführten Finger-Ziehbewegungen können aufgezeichnet und es kann daraus ein Bewegungsprofil erstellt werden. Die Auswertung auf Gleichförmigkeit und Geradlinigkeit der Bewegung, Aussetzen, Tempo, Länge, Druckstärke, verstrichene Zeit zwischen den Umkehrpunkten erlaubt Rückschlüsse auf das jeweilige Entspannungsniveau der übenden Person.

Es ist weiters eine Echtzeit-Berechnung des momentanen Atemrhythmus möglich, sodass ein gezieltes Starten der Sprachausgabe zum Zeitpunkt des Einatmens oder des Ausatmens nach trainingsmethodischen Erfordernissen erfolgen kann (wenn die übende Person z.B. eine Aussage mit bestimmter Information in sich "aufnehmen" soll, so erfolgt die Ansage gezielt zu Beginn des Einatmens; wenn der beabsichtige Inhalt der Aussage z.B. "locker lassen" ist, so erfolgt die Ansage gezielt zu Beginn des Ausatmens).

Es ist die Erkennung von Abdriften der Konzentration oder von unerwünschtem Einschlafen aufgrund des Aussetzens der Ziehbewegungen möglich. Eine darauf angemessene Steuerung des Programmablaufs könnte so erfolgen, dass die Sprachansage und/öder ein Weckgeräusch nach Ablauf eines vordefinierten Zeitintervalls ohne Ziehbewegungen (Anwendung des Prinzips der Totmanntaste für Lokführer in einem hier gänzlich anderen Kontext) immer lauter werdend abgespielt wird.

Es kann auf einen ansonsten erforderlichen externen Sensor (z.B. einen Brustgurt zur Messung des Atemrhythmus) verzichtet werden. Dies erspart nicht nur dessen Anschaffung, sondern auch den Aufwand seines Anbringens samt Vermeidung möglicher Fehler bei falschem Anbringen sowie das unangenehme, beengende Gefühl, welches derartige zur Messung angebrachte Sensoren bei der übenden Person erzeugen.

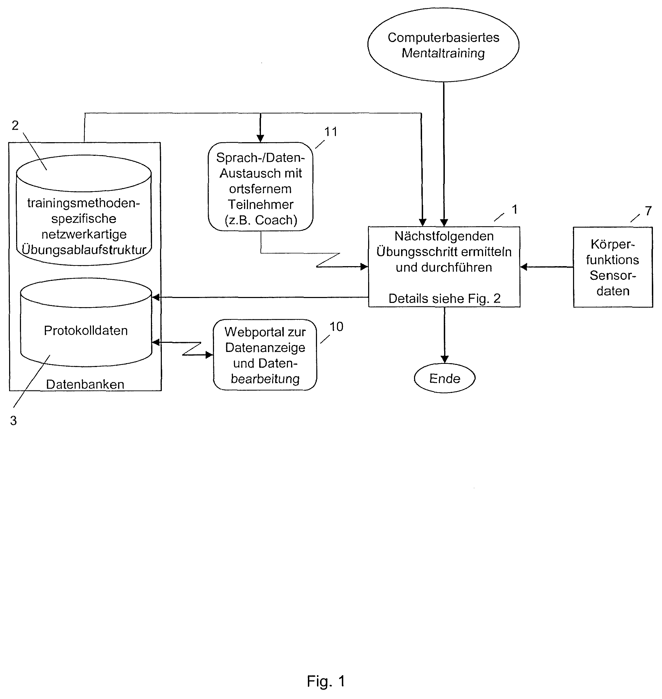

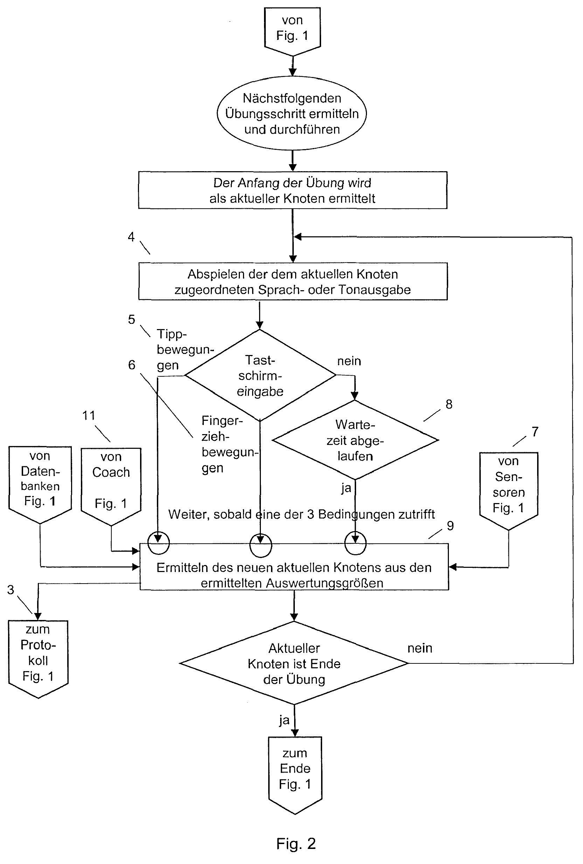

Die Erfindung wird nachfolgend anhand eines in der Zeichnung schematisch dargestellten Beispiels näher erläutert. In dieser zeigen Fig. 1 den Hauptprozessablauf des erfindungsgemäßen Verfahrens und Fig. 2 den Prozessschritt der Ermittlung des nächstfolgenden Programmschritts im Programmablauf.

In Fig. 1 wird das erfindungsgemäße Verfahren anhand eines Beispiels für computerbasiertes Mentaltraining erläutert. Das computerimplementierte Verfahren greift auf die Datenbank 2 zu, in der eine trainingsmethodenspezifische netzwerkartige Übungsablaufstruktur gespeichert ist. Die Übungsablaufstruktur enthält alle möglichen Trainingsschritte des Trainingsprogramms, wobei zumindest einigen der Trainingsschritte oder allen Trainingsschritten eine elektronisch gespeicherte Sprachansage oder Tonfolge zugeordnet ist. In der Übungsablaufstruktur können die einzelnen Trainingsschritte ein einer hierarchischen Struktur, wie z.B. in einer Baumstruktur, dargestellt sein. Dadurch wird eine bestimmte Reihenfolge der Trainingsschritte vorgegeben, weil die Durchführung eines in der Hierarchie untergeordneten Trainingsschritts die vorherige Durchführung eines in der Hierarchie übergeordneten Trainingsschritts voraussetzt. Die Übungsablaufstruktur ist hierbei so ausgebildet, dass an einer Reihe von Knotenpunkten der Struktur hinsichtlich des als nächstes durchzuführenden Trainingsschritts eine Wahlmöglichkeit besteht.

Mit dem erfindungsgemäßen Verfahren soll nun ein Programmablauf bestimmt werden, d.h. der Zeitpunkt und die Reihenfolge der Durchführung der in der Übungsablaufstruktur vorgesehenen Trainingsschritte. Zu diesem Zweck wird die in der Datenbank 2 gespeicherte Übungsablaufstruktur in einem schematisch mit 1 bezeichneten Verfahrensschritt herangezogen, um den nächstfolgenden Trainingsschritt zu ermittelt und durchzuführen. Die Durchführung des nächstfolgenden Trainingsschritts bedeutet im Falle eines aus dem Abspielen einer Sprachansage oder einer Tonfolge bestehenden Trainingsschrittes, dass diese Sprachansage oder Tonfolge von einer geeigneten Hardwarekomponente des elektronischen Geräts, auf welchem das Verfahren durchgeführt wird, abgespielt wird. Bei der Ermittlung des nächstfolgenden Trainingsschrittes kann zusätzlich ein Sprach- bzw. Datenaustausch mit einem ortsfernen Trainer (z.B. Coach) gemäß Schritt 11 vorgenommen werden, wobei der Trainer in den Programmablauf eingreifen kann. Außerdem können bei der Ermittlung des nächstfolgenden Trainingsschrittes auch Körperfunktionsdaten 8, die mit Hilfe geeigneter Sensoren aufgenommen werden, berücksichtigt werden.

Der Programmablauf wird in Form von Protokolldaten 3 in einer Datenbank gespeichert, was eine nachträgliche Analyse und Auswertung des Trainingsprogramms erlaubt, sodass z.B. Trainingsfortschritte festgestellt werden können. Der Zugriff auf die Protokolldaten kann über ein Webportal 10 zur Datenanzeige und ggf. Datenbearbeitung erfolgen.

In Fig. 2 sind die Detailabläufe bei der Ermittlung des nächstfolgenden Trainingsschrittes dargestellt. Ausgangspunkt ist der aktuelle Knoten der generellen Übungsablaufstruktur 2, an dem sich der Programmablauf befindet. Am aktuellen Knoten wird die diesem Knoten zugeordnete Sprachansage oder Tonfolge ausgegeben (Schritt 4). Zur Ermittlung des nächsten Trainingsschrittes werden Benutzereingaben, einschließlich periodischer Bewegungen auf einer berührungssensitiven Eingabeoberfläche erfasst und ausgewertet. Die Benutzereingabe kann eine vorzugsweise periodische Tippbewegung 5 oder eine Fingerwisch- bzw. -ziehbewegung 6 sein. Im Schritt 9 wird in Abhängigkeit von der ermittelten Benutzereingabe und deren Auswertung oder nach Ablauf einer vorgegebenen Wartezeit 8 ohne Benutzereingabe der nächste Trainingsschritt ermittelt und dadurch der Programmablauf gesteuert. Die Ermittlung des nächsten Trainingsschrittes umfasst dabei die Ermittlung eines neuen Knotens aus der generellen Übungsablaufstruktur 2. Wenn der neue Knoten der letzte Knoten in der Übungsablaufstruktur 2 ist, wird das Trainingsprogramm beendet. Andernfalls wird der in Fig. 2 beschriebene Ablauf wiederholt.

Eine wesentliche Stärke des computerbasierten Mentaltrainings ist sein Wirkkreislauf zwischen den vom Verfahren passend zu den jeweiligen Übungszielen sowie zur momentanen Trainingssituation aus zeitnahen sowie historischen Daten 2,3 ermittelten (Schritt 1) sowie den aktuellen mentalen Zustand der übenden Person bestmöglich berücksichtigend ausgegebenen (Schritt 4) Übungsanweisungen und den als direkte Auswirkung darauf von der Person gezeigten bewussten Reaktionen durch Tipp-Bewegungen 5, sowie den auf Eigenbeobachtung basierenden Reaktionen, aufgezeichnet durch Ziehbewegungen 6 oder durch Messungen unterschiedlicher bekannter Körperfunktionssensoren 7, woraufhin sämtliche dermaßen erhobenen Daten in die Ermittlung des nächstfolgenden Übungsschritts 9 mit einbezogen werden und wahlweise zusätzlich auch direkt an die Person rückübermittelt werden (Schritt 4).

Eine vorteilhafte Ausgestaltung stellt die Möglichkeit dar, die Eigenwahrnehmungsmessung einer Körperfunktion mittels Ziehbewegungen 6 durchzuführen, welche während jeder bereits laufenden Übung durch einfache Handhabung ergänzend durchführbar ist, ohne irgendwelche Zusatzgeräte zu erfordern und ohne zusätzliche Übungszeit zu beanspruchen. Objekt der Eigenwahrnehmung ist vorzugsweise der eigene Atemverlauf. Die Atmung ist die einzige menschliche Körperfunktion, die genau an der Schwelle zwischen kognitivem und vegetativem Nervensystem arbeitet. Atmung kann daher sowohl völlig bewusst von uns wahrgenommen und beeinflusst werden, sie wird aber die meiste Zeit hindurch gänzlich und ohne unsere bewusste Wahrnehmung ausschließlich vom vegetativen System gesteuert. Das Üben eines intensivierten Bewusstseins für die eigene Atemwahrnehmung ist deshalb ein bei allen anerkannten Mentaltrainingsmethoden angewendeter Zugang zur Erreichung eines für Mentalübungen bestgeeigneten, entspannten und assoziationsbereiten Zustands.

Das Verfahren fördert und kontrolliert bewusstes Atmen, indem es die übende Person dazu anleitet, zeitgleich mit ihren eigenen Atemvorgängen gleichlaufende Eingabebewegungen 6 auszuführen, vorzugsweise den Finger zur Person hin ziehen während des Einatmens und von der Person weg ziehen während des Ausatmens, jeweils mit demselben Finger am Tastbildschirm eines Smartphones oder eines Tablet PCs, oder am Touchpad oder mit der Maus eines PCs ausgeführt.

Das Verfahren macht sich dabei das bekannte Lernen durch motorische Unterstützung zunutze. So wie auch beim handschriftlichen Schreiben, bei dem die Aufmerksamkeit und in weiterer Folge die Merkfähigkeit für die geschriebenen Inhalte durch die motorisch koordiniert auszuführenden Bewegungen der Hand stärker gefördert werden, als es nach zeitgleichem bloßem Aufsagen oder nur gedanklichem Vergegenwärtigen derselben Inhalte der Fall wäre, so führen auch die kontinuierlichen Wischbewegungen zu einer vertieften und bewussteren Atemwahrnehmung.

Einmal begonnen, wird das Ausführen der atembegleitenden Bewegungen von der übenden Person beständig fortgesetzt, während auch die ursprüngliche Übung weiterläuft. Nur zur Ausführung der zur Ablaufsteuerung gegebenenfalls erforderlichen Tippbewegungen 5 werden die Ziehbewegungen kurz unterbrochen und sofort danach wieder aufgenommen. Dadurch können sowohl die ursprüngliche Übung als auch die Eigenwahrnehmungsmessung zeitgleich und unabhängig voneinander ablaufen und von der übenden Person mit demselben Finger und ohne Erfordernis eines Blickkontakts ausgeführt werden. Falls erwartete atembegleitende Ziehbewegungen 6 länger als für eine vordefinierte Wartezeit 8 nicht oder nicht mehr korrekt ausgeführt werden, so wird als nächste Ausgabe 4 eine Erinnerungsanweisung abgespielt, wodurch das Abgleiten der Aufmerksamkeit bis hin zu einem unerwünschten Einschlafen während des Übens zeitnahe erkannt und somit höchst wirksam verhindert werden kann.

Aus den Ziehbewegungsdaten wird über einen vorab definierten Zeitverlauf hinweg das Ausmaß an Gleichförmigkeit und Regelmäßigkeit ermittelt, wobei ein hohes Ausmaß als tiefere Entspannung interpretiert wird und umgekehrt. Die so ermittelten und gespeicherten 3 Werte fließen in die Ermittlung des nächstfolgenden Trainingsschritts 9 mit ein.

Eine weitere vorteilhafte Ausgestaltung stellt die Möglichkeit dar, einen oder mehrere bekannte Körperfunktionssensoren 7 während der laufenden Übung an der übenden Person zur Messung anzubringen. Die jeweils zeitnah ermittelten Werte fließen in die Ermittlung des nächstfolgenden Übungsschritts 1 mit ein.

Eine weitere vorteilhafte Ausgestaltung stellt die Möglichkeit dar, während der laufenden Übung und ohne Unterbrechung des Übungsablaufs rückgekoppelte Informationen zum Werteverlauf der aktuell ermittelten Daten zur Eigenwahrnehmungsmessung 6 oder aus Messwerten von Körperfunktionssensoren 7 durch Sprachansagen und/oder Tonsignale auszugeben. Durch dieses auditive Biofeedback kann die übende Person zeitnahe zuordnen und lernen, welche ihrer Verhaltensweisen zu welchen mentalen Zuständen führen und kann so ein rascheres und verlässlicheres Hervorrufen und Beibehalten eines von ihr erwünschten Zustands wirksamer trainieren.

Eine weitere vorteilhafte Ausgestaltung stellt die Möglichkeit dar, anstelle von Tippbewegungen am Tastbildschirm oder Klickbewegungen mit der Maus andere Möglichkeiten der Benutzerrückmeldung zu verwenden. Um dabei eine möglichst konsistente Benutzerschnittstelle zu gewährleisten, ist bei allen, in ihrer Ausrichtung und in ihrem Zweck logisch zusammengehörenden Auswahlmöglichkeiten die Verwendung derselben Auswahlzahl vorzusehen. Dabei sollen vorzugsweise Auswahlmöglichkeiten mit Reihenfolgenummer zwei für zustimmende oder den momentanten Übungsablauf fortführende Rückmeldungen, mit Reihenfolgenummer drei für ablehnende oder den Übungsablauf unterbrechende Rückmeldungen oder zur Anforderung zusätzlicher Hilfe-Information, mit Reihenfolgenummer vier für Auflistungen zusätzlicher Auswahloptionen, mit Reihenfolgenummer fünf für die Rückkehr entweder zur zuvor ausgeführten Aktion oder zum zuletzt ausgeführten Übungsschritt verwendet werden.

Eine weitere vorteilhafte Ausgestaltung stellt die Möglichkeit dar, bei Ausgabe von Sprachansagen 4 zufallsgesteuert durch Sprechtempoänderungen, Sprechpausen oder sprechtypische Begleitgeräusche wie etwa Atmen oder Räuspern, einen möglichst lebensnahen- Eindruck der ansagenden Stimme zu vermitteln. Dadurch wird die für den Übungserfolg wichtige Bereitschaft der übenden Person, sich möglichst unvoreingenommen und irritationsfrei auf die Übung einzulassen, zusätzlich unterstützt.

Das Ziel einer möglichst hohen Akzeptanz und Nutzungsbereitschaft für das Verfahren wird dadurch erreicht, dass es etwa als am Smartphone verfügbar gemachte App immer zur Hand ist und ohne umfangreiche Vorbereitungen zum jederzeitigen Üben sofort einsatzbereit ist. Eine weitere vorteilhafte Ausgestaltung stellt deshalb die Möglichkeit dar, eine einstellbare Erinnerungsfunktion einzurichten, die auf Wunsch entweder termingesteuert oder zu zufälligen Zeitpunkten auf die jeweils bestgeeignetste nächste durchzuführende Übungseinheit hinweist, woraufhin diese auf Wunsch mittels entsprechender Eingabe 5 auch sofort gestartet werden kann.

Eine weitere vorteilhafte Ausgestaltung stellt die Möglichkeit dar zu messen, wie weit es gelingt, im eigenen Tagesablauf Zeiträume der Ruhe und des Abschaltens zu reservieren, tatsächlich einzuhalten oder sogar auszudehnen. Dazu definiert die übende Person beliebige Zeitintervalle, zu denen eine zusätzliche Protokollierung 3 sämtlicher Aktivitäten auf dem verwendeten Gerät, vorzugsweise einem Smartphone, durchgeführt wird. Dazu werden alle durchgeführten Aktivitäten wie Telefonate, Internetzugriffe oder Programmaufrufe gruppenweise gezählt und ihre jeweils benötigten Ausführungszeiten gruppenweise summiert. Die Aktivitäten werden dabei nur mengenmäßig erfasst, Zugriff oder gar Speichern von konkreten Inhalten erfolgt nicht. Die übende Person erhält dadurch einen Überblick über ihr Nutzungsverhalten, kann geplante Nutzungspausen vorsehen oder ein gewünschtes Nutzungsverhalten als Ziel definieren und kann ihre Zielverfolgung über beliebige Zeiträume hinweg verfolgen.

Für die Nutzung sämtlicher bisher angeführten Ausgestaltungen benötigt die Durchführung des Verfahrens selbst keine Datenfernverbindung. Wenn also nur die bis jetzt beschriebenen Funktionalitäten benötigt werden, dann genügt zur Anwendung ein marktübliches Smartphone im sogenannten Offline Modus. Vorteilhaft fallen hierbei keine laufenden Kosten an, sämtliche Übungsdaten sind sicher vor unerwünschter oder unbeabsichtigter elektronischer Weitergabe und es kann keine Mobilfunkstrahlung auftreten.

Die folgenden vorteilhaften Ausgestaltungen nutzen zusätzlich auch die auf Smartphones oder auf Computern vorhandenen Möglichkeiten zum Aufbau von Sprach- oder Datenfernverbindungen.

Eine dieser vorteilhaften Ausgestaltungen stellt die Möglichkeit dar, eigene Trainingsprotokollinformationen und dazugehörige Kommentare in ein dafür bereitgestelltes Webportal im Internet 10 hochzuladen. Durch diese Möglichkeit zum Austausch können Wissen, Motivation und Übungsbereitschaft in einer Gruppe von jeweils einzeln übenden Personen gesteigert werden.

Eine weitere vorteilhafte Ausgestaltung stellt die Möglichkeit dar, vor oder während des Verlaufs einer Übung Kontakt zu einer oder zu mehreren ortsfernen Teilnehmern, typischerweise zu einem Coach oder Mentaltrainer 11 herzustellen, wodurch nicht nur sämtliche jeweils aktuell bei der übenden Person vorhandenen Daten und Audiosignale 2, 3, 4 mitverfolgt oder überprüft werden können, sondern bei Bedarf auch in die laufende Übungsschrittermittlung 9 steuernd eingegriffen oder in direkte Sprechoder Chatverbindung 11 mit der übenden Person getreten werden kann.

## Patentansprüche

1. Computerimplementiertes Verfahren zur Steuerung des Programmablaufs eines Trainingsprogramms, insbesondere für mentales Training, bei welchem der Programmablauf den Zeitpunkt und die Reihenfolge des Abspielens von elektronisch gespeicherten Sprachansagen oder Tonfolgen bestimmt, **dadurch gekennzeichnet, dass** periodische Bewegungen des Benutzers auf einer berührungssensitiven Oberfläche erfasst und ausgewertet werden und der Programmablauf in Abhängigkeit vom Ergebnis der Auswertung gesteuert wird.

2. Verfahren nach Anspruch 1, **dadurch gekennzeichnet, dass** die periodische Bewegung eine hin und hergehende Wisch- oder Ziehbewegung ist.

3. Verfahren nach Anspruch 1 oder 2, **dadurch gekennzeichnet, dass** die periodische Bewegung synchron mit dem Herzschlag oder der Atemfrequenz des Benutzers erfolgt.

4. Verfahren nach Anspruch 1, 2 oder 3, **dadurch gekennzeichnet, dass** die periodischen Bewegungen durch eine berührungssensitive elektronische Anzeigevorrichtung, insbesondere ein Touchdisplay, eines Computers, wie z.B. eines SmartPhones, Tabletcomputers oder Personal Digital Assistent (PDA), erfasst werden.

5. Verfahren nach einem der Ansprüche 1 bis 4, **dadurch gekennzeichnet, dass** das Auswerten der periodischen Bewegungen die Ermittlung einer Auswertungsgröße umfasst, wobei die Steuerung des Programmablaufs in Abhängigkeit von der Auswertungsgröße erfolgt.

6. Verfahren nach Anspruch 5, **dadurch gekennzeichnet, dass** als Auswertungsgröße die Häufigkeit der Bewegungen, die Anzahl von Bewegungen, die Druckstärke, die Geschwindigkeit der Bewegungen, die Frequenz der periodischen Bewegung, insbesondere der Hin- und Herbewegung, die Regelmäßigkeit der Bewegungen und/oder der zeitliche Abstand zwischen zwei aufeinanderfolgenden Bewegungen ermittelt wird.

7. Verfahren nach Anspruch 5 oder 6, **dadurch gekennzeichnet, dass** die Auswertungsgröße kontinuierlich oder in regelmäßigen Abständen ermittelt wird und mit wenigstens einem vorgegebenen Grenzwert verglichen wird, wobei eine dem wenigstens einen vorgegebenen Grenzwert zugeordnete, gespeicherte Sprachansage oder ein Tonsignal bei Erreichen oder Unter- bzw. Überschreiten des Grenzwerts abgespielt wird.

8. Verfahren nach Anspruch 5, 6 oder 7, **dadurch gekennzeichnet, dass** die Wiedergabegeschwindigkeit der Sprachansage in Abhängigkeit von der ermittelten Auswertungsgröße variiert wird.

9. Verfahren nach einem der Ansprüche 5 bis 8, **dadurch gekennzeichnet, dass** die Pausenlänge zwischen dem Abspielen zweier aufeinanderfolgender Sprachansagen in Abhängigkeit von der ermittelten Auswertungsgröße gewählt wird.

10. Verfahren nach einem der Ansprüche 5 bis 9, **dadurch gekennzeichnet, dass** das Abspielen einer Sprachansage im Wesentlichen gleichzeitig mit dem Beginn der erfassten Bewegung gestartet wird.

11. Computerprogrammprodukt umfassend Programmcode-Mittel geeignet zur Durchführung der Schritte eines Verfahrens nach einem der Ansprüche 1 bis 10, wenn das Computerprogrammprodukt auf einer Recheneinrichtung ausgeführt wird.

12. Recheneinrichtung, insbesondere Smartphone oder Tabletcomputer, zur Durchführung des Verfahrens nach einem der Ansprüche 1 bis 10, wobei die Recheneinrichtung eine berührungssensitive Oberfläche aufweist, **dadurch gekennzeichnet, dass** die Recheneinrichtung mit einem Computerprogrammprodukt nach Anspruch 11 programmiert ist.
